# EUROPEAN PATENT APPLICATION

(11) **EP 2 317 368 A1**
(43) Date of publication of application: **04.05.2011**
(21) Application number: 09761831.8
(22) Date of filing: 03.06.2009
(51) Int. Cl.: G02C 7/10

(54) **DEVICE FOR ASSISTING AND PROTECTING VISION**

(30) Priority: 11.06.2008 ES 200801761
(71) Applicant: Universidad de VAlladolid, 47002 Valladolid (ES); Fundacion Cidetec, 20009 San Sebastián (Guipúzcoa) (ES); Universidad Carlos III De Madrid, 28911 Leganes (Madrid) (ES)
(72) Inventor: COCO MARTIN, Rosa Maria, E-47002 Valladolid (ES); CUADRADO ASENSIO, Rubén, E-47002 Valladolid (ES); COCO MARTIN, Maria Begoña, E-47002 Valladolid (ES); ALBERTO LÁZARO, José, E-47002 Valladolid (ES); POZO GONZALO, Cristina, E-20009 San Sebastian (Guipúzcoa) (ES); SALSAMENDI TELLERIA, Maitane, E-20009 San Sebastian (Guipúzcoa) (ES); POMPOSO ALONSO, José Adolfo, E-20009 San Sebastian (Guipúzcoa) (ES); GRANDE TELLERIA, Hans-Jurgen, E-20009 San Sebastian (Guipúzcoa) (ES); VERGAZ BENITO, Ricardo, E-28911 Leganes (Madrid) (ES); TORRES ZAFRA, Juan Carlos, E-28911 Leganés (Madrid) (ES); BARRIOS PUERTO, David, E-28911 Leganés (Madrid) (ES); SÁNCHEZ PENA, Jose Manuel, E-28911 Leganés (Madrid) (ES)
(74) Representative: Ungria Lopez, Javier
(86) International application number: PCT/ES2009/070202
(87) International publication number: WO 2009/150274

(57) **Abstract**

The invention relates to a device comprising one left electrochromic filter (3) and one right electrochromic filter (4) controlled by an electronic device (12). Said electronic device has means for controlling at least one filtering parameter, said means being actuatable by the user and programmed by a professional ophthalmologist, or a combination of the two. The aim of the invention is to provide variable filtering for the vision of a user of the device.

## Description

### OBJECT OF THE INVENTION

The present invention, as expressed in the title of this specification, is related to a device for assisting and protecting vision, the essential purpose of which consists of providing a device that improves vision of users with disabilities such as lack of adaptation to light/dark changes, photophobia, low vision, high sensibility to glare, or other, the device of the invention further providing protection against short wave radiation (lower than 450 nm), for which the invention uses electrochromic mixtures able to vary the spectral transmittance optical properties as a response to an electric current, and the device of the invention being able to be achieved with an appearance similar to that of standard glasses- The present invention is framed within the technical field of aids for patients with low vision, and more particularly in that of selective absorption filters.

### BACKGROUND OF THE INVENTION

According to the WHO there is low vision (LV) in those subjects with corrected visual acuity ≤ 0.3 in the better eye even after treatment and/or ordinary refraction, where there is a visual residue potentially usable for tasks' planning and execution. This people with LV often use selective absorption filters in the form of tinted glasses as an attempt to improve their visual function and also their comfort.

A filter is a device that modifies the spectral distribution of the light reaching the eye. Several studies have assessed the effect of the filters absorbing short wave lengths (often called blue blockers) in patients with LV. Some have found a subjective preference for yellows and oranges (Hoeft and Hughes, 1981; Morrissette et al, 1984; Maino and McMahon 1986; Provines et al, 1997), while others have shown objective improvements in visual function (Hellinger, 1983; Lynch and Brilliant, 1984; Tupper et al., 1985; Maino and McMahon, 1986; Zigman, 1990; Leat et al., 1990; Frennesson and Nilsson, 1993; Rosenblum et al., 2000; Wolffsohn et al., 2002; Eperjesi et al., 2004; Leat et al., 1994; Hazle et al., 2000; McClure et al., 2000).

According to Millodot (Millodot, Dictionary of Optometry and Visual Science, Butterworth-Heinemann, Oxford. 1999) there is Glare when the existing illumination has an adverse effect on comfort or visual resolution. Filtration by absorption lens is an effective method for extracting short wave (280-400 nm) ultraviolet, violet and blue radiation, from the emission spectra of light sources, which seems to effectively reduce the glare. It must be taken into account that in the 500 nm region the attenuation of the radiation also starts to negatively affect vision (Rosenbert, Chapter 10, In: Faye ed, Little Brown and Company Inc, Boston, 1984).

The problems of adaptation to darkness imply an exaggerated sensibility to light after being in the darkness. It seems to be shown that the CPF550® and the NOIR® filters improve the adaptation to dark (Lynch and Brillant 1984). It also seems that the adaptation to dark is accelerated when wearing filter during pre-adaptation (Van den Berg 1990). Moreover, the sensibility to contrast is one of the best predictors of mobility and it frequently improves with filters (Marron & Bailey, 1982). One additional advantage of filtration by absorption lenses is the elimination of Scattering by reducing deviated radiation (especially short wave length) entering the eye from peripheral areas and which randomly reflects in its interior (Rosenberg, Chapter 10, In: Faye ed, Little Brown and Company Inc., Boston, 1984).

Moreover, filters are specially useful in opacities of the means (e.g.: cataract) since in these cases the ultraviolet and blue light diffusion can produce a veil luminance on the retinal image, which causes a contrast reduction on it (Tupper et al., 1985; Steetn et al.,1993: Sakamoto, 2002). Moreover, the absorption of short wave lengths with yellow or orange filters seems to improve the contrast of the retinal image in the aged eye (Rosenberg, Chapter 10, In: Faye ed., Little Brown and Company Inc., Boston, 1984).

The works of Adrian and Schmidt (1977) which showed that retinal damage in mice with Pigmentary Retinosis decreased and slowed down when brown filters were used are classic. In any case, it is known that most subjects with Retinosis improve their sensibility to contrast with filters when there are high levels of luminance (Wetzel et al, 1996). Young (1993, 1994) has also advocated for the theory that sunlight is an important causal factor of some eye diseases highly prevalent as ARMD, senile cataract, pterygium, photokeratitis and eyelid cancer.

With respect to the currently commercially available lenses, photochromatic lenses generally have the inconvenience of taking several minutes for changing coloring (Nowakowsky, Primary low vision care, Appletton & Lange, Norwalk, 1994). Moreover, the prescription of ophthalmic filters should be personalized The lens with which he/she is really comfortable in different illumination conditions should be tested on each patient and what we frequently find is that our patients require at least two different filters, one for indoors and another one for outdoors. That is why, having a unique personalized filter the characteristics of which could change from one to the other in a few seconds would be a major advantage for these patients.

Currently electrochromic materials for sunglasses with high optic transmittance in its clear state have been developed, but selective absorption filters that eliminate wave lengths deleterious for the eye have not been developed in any of the previously explained senses.

In 1993 Nikon marketed some Ecs glasses, but since then, and observing the response in the market, there has been little movement in that respect, since the price is still excessive. In fact, Nikon withdrew them from the market (Granqvist, 2000). The advantages of using electrochromism in glasses are obvious: the same glasses may serve for environments with different illumination conditions. Although photochromics already do this the difference is that electrochromic materials commute more rapidly and, above all, in a controlled manner. Unfortunately, during the 90's the technology was still expensive and the drivers could be costly. However, there has been a more recent review on this matter. The company Dynamir, subsidiary spin-out from Ashwin-Ushas' activity on electrochromic materials, has developed at least some prototypes that seem to work reasonably well, also using a sensor system for detecting the environmental illumination. However, it continues to be a technology too expensive for the market (testing devices are rented for 60 days for approximately 300 dollars; in other scopes it is noted that the price of some Ecs glasses may be around 1200 dollars). Research continues in order to find both more affordable materials and more specific applications (ocular protection needs in the industry, patients with macular degeneration, low vision, just to name a few examples).

On the other hand, ophthalmologic filters prepared from an etectrochromic composition, such as the one described on Patent P-200800258 (property of CIDETEC), mainly made of an electrochromic material derived from viologens, a polymeric matrix, an electronic mediator allowing the re-dox reaction, a plasticizer and a suitable solvents mixture that dissolves the aforementioned products are known. Said filters have the characteristics that both the electrochromic composition and the assembly of the device are prepared at room temperature, providing simplicity to the manufacturing process.

### DESCRIPTION OF THE INVENTION

In order to achieve the objectives and avoiding the drawbacks mentioned in the previous paragraphs, the invention consists of a device for assisting and protecting vision, comprising one left electrochromic filter and one right electrochromic filter governed by an electronic device.

In a novel manner, according to the invention, the referred device presents means for controlling at least one filtering parameter, actuatable by the user, programmable by a professional ophthalmologist or a combination of the two, with the object of establishing variable filtering of the spectral transmittance in the vision of a user of the device.

According to the preferred embodiments of the invention, the referred variable filtering is established for the absorption of short wave lengths in at least the 400 to 550 nm band.

According to the preferred embodiments of the invention, said assisting device is optionally achieved combined with a standard optical correction element such as some graduated glasses.

The assisting device of the invention can consist of a series of supplements that are coupled to those graduated glasses,

On the other hand, the electronic device previously mentioned can be included in one of the side pieces of a glasses' body wherein the areas of the frame that are in front of the eyes of the user include the referred electrochromic filters.

According to the preferred embodiment of the invention, the electronic device that has been mentioned presents supply means and has a D/A converter the output of which connects with the electrochromic filters, while a memory and a control circuit are connected to its input which through an A/D converter connects with an external potentiometer and through a connection module couples to a connector or external terminal; such that this terminal forms the referred programmable means and the potentiometer forms the above-mentioned means actuatable by the user.

The referred memory can be an EPROM memory, the control circuit can be a PIC circuit or a general purpose microcontroller, the connection module can be an RS-232-type and the external terminal can be a USB-type.

The above-mentioned supply means can consist of a rechargeable battery located in a belt of the user and cable-connected to the rest of the electronic device, or else it can consist of a button cell located in the frame of the corresponding glasses.

On the other hand, the external potentiometer can consist of a variable resistor of sliding or rotating control, or else of two buttons with associated electronic circuitry respectively facilitating an increase and decrease of the filtering carried out by the electrochromic filters.

The above-mentioned external terminal forming the programmable means is capable of being complemented or substituted by infrared or radiofrequency wireless programming means applicable to the control circuit.

According to the preferred embodiments of the invention, the device of the invention has feedback means applied to the electronic device and determined by photodiodes located in front and behind the electrochromic filters.

The device of the invention can be provided with a programming differencing the type of environmental illumination using the signs of the referred photodiodes, consequently acting on the electrochromic filters.

On the other hand, said device can have voltage wave forms exciting the electrochromic filters specifically adapted to its operation, continuous or alternate, these being square with variable work cycle or triangular or sinusoidal, with the object of optimizing the current draw of said filters.

Moreover, according to different embodiments, the referred control means of the device of the invention are capable of being achieved in such a way that they independently act on the left electrochromic filter and on the right electrochromic filter, or else in such a way that they act jointly on both of them.

It is appropriate to note that the change determined by the filtering in the referred spectral band is not on the complete level, but with spectral variations, and that the support over which the electrochromic material is synthesized already eliminates all the ultraviolet range, up to 400 nm, this value being therefore justified. In order to justify reaching to 550 nm, is enough to say that if it was filtered in higher wave lengths a great part of the visual function would be lost. The interest for filtering the band below 550 nm in a spectrally selective manner is that it improves the visual function by eliminating radiation of wave lengths causing scattering in the eye, glares and veil luminance in the retina in persons with low vision, further protecting the eye from generally deleterious radiations.

With the described structure, the device of the invention has advantages in that it facilitates a fast and voluntary selection of the filtering made by the glasses of the user, allowing applications in patients with low vision, protecting the eyes from short wave radiations and facilitating programming by professional ophthalmologists in the filtering of the spectral transmittance that is going to be made, such that the device can be personalized. Moreover, said device can be combined with a standard optical graduation.

Next, in order to facilitate a better understanding of this specification and forming and integral part thereof, some figures are attached wherein with an illustrative but not limitative character the object of the invention has been represented.

### BRIEF DESCRIPTION OF THE FIGURES

**Figure 1****.-** Represents a schematic block diagram of a device for assisting and protecting vision made according to the present invention.
**Figure 2****.-** Represents a perspective view of some glasses including the device previously mentioned in figure 1.

### DESCRIPTION OF AN EXAMPLE OF EMBODIMENT OF THE INVENTION

In the following a description of an example of the invention referring to the numbering adopted in the figures is made.

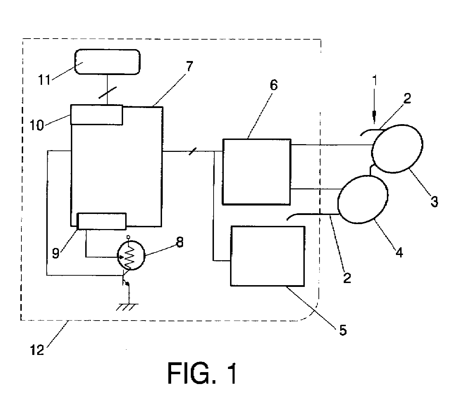

Thus, the device of the present example comprises one left electrochromic filter 3 and one right electrochromic filter 4 governed by an electronic device 12, as it can be seen in figure 1.

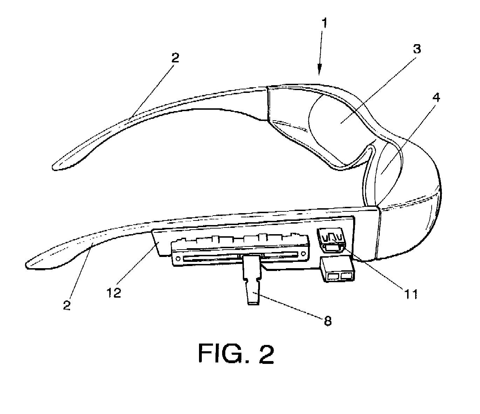

Said electronic device 12 is included in one of the sidepieces 2 of some glasses 1, as it can be seen in figure 2.

On the other hand, as it can be appreciated in figure 1, the electronic device 12 includes a digital/analog converter 6 which connects with the filters 3 and 4 and in the input of which an EPROM 5 memory and a PIC control circuit 7 are arranged.

Said control circuit 7 connects with an external potentiometer 8 through an analog/digital converter 9; further connecting the control circuit 7 through an RS-232 connection module referenced as 10 with a USB-type terminal connector 11, all of it as it is represented in figure 1.

With this some filtering selection means actuatable by the user through the potentiometer 8 and programmable by a professional ophthalmologist through the external terminal 11 are established.

The device of the present example is able to rapidly change (in the range of seconds) its selective spectral filtration characteristics as required by the patient.

The originality of the present Invention resides in the application of a new type of filtrating device able to change its optical properties in the presence of a variation in the electric potential applied thereon, which will allow controlling the transmittance of the light by the filter developed with him at the patient's will in terms of the conditions of environmental illumination wherein he/she is at, this change being able to be produced in a few seconds. The most important application of the present invention is the use of these electrochromic ophthalmic filters as an aid for low vision. That is why we will move in the range of between 400 and 550 nm as the lower limit of the transmission spectral range.

With respect to the electrochromic device, according to the present example ophthalmologic filters prepared from an electrochromic composition described in Patent P-200800258 are used, mentioned under the section backgrounds of the invention, mainly formed by an electrochromic material derived from viologens, a polymeric matrix, an electronic mediator allowing the re-dox reaction, a plasticizer and a suitable mixture of solvents that dissolves the aforementioned products. These filters are characterized in that both the electrochromic composition and the assembly of the corresponding device are prepared at room temperature, providing simplicity to the manufacturing process. The electrochromic composition was applied on a conductive clear substrate highly transparent by techniques traditionally used in painting deposition including spin-coating, doctor blade or dip-coating. Glass spacers with a diameter of 1.5 micron were added, for a uniform and optimal deposition. The conductive transparent substrates are based on plastic or crystal, crystal substrates being preferable for favoring the transparency in low vision patients and obtaining optical quality. Finally, with respect to the electrochromic device, a sealing using an epoxy resin curable by ultraviolet was carried out.

With respect to the control electronics, a control electronic circuit was designed and implemented with stress applied to the electrochromic devices or filters 3 and 4, which was arranged in the temple 2 of the glasses 1, as it can be appreciated in figure 2. The applied tension is proportional to the transmittance of the crystals. The linear potentiometer 8 allows selecting the level of tension by the user. The maximum and minimum levels will depend on the ophthalmologic particularities of the user. Specific software allowing the professional ophthalmologist testing and subsequently programming these tension levels, according to the indications of the patient, has been developed. On the other hand, a feedback is used in the system, such that photodiodes anterior and posterior to the crystals offer the levels of radiation, their relation being the transmission of the crystals. Owing to that, a feedback protocol via software is established with which real time control of the radiation reaching the eye is achieved, in terms of the intensity of the environmental illumination, as well as of the spectral characteristics of the same, The supply of the electronic device 12, according to the present example, can consist of a traditional rechargeable battery implemented in a coat wallet or localizable in a belt of the user, which is coupled to the glasses by a cable, being able to also consist of one or two button cells arranged in the temples 2 of the glasses 1; the selection of one or the other option depending on the user and on the current needs.

The device of the present example shows a maximum optical contrast of 80% and commutation times of 3 and 5.6 seconds during the coloration and discoloration processes, respectively. Moreover, the device of the present example has been subjected to cyclability studies (above 2000 cycles) with very satisfying results, its range of transmittance variation decreasing in less than 10%.

## Claims

1. DEVICE FOR ASSISTING AND PROTECTING VISION, comprising one left electrochromic filter (3) and one right electrochromic filter (4) governed by an electronic device (12); **characterized in that** it presents control means of at least one filtering parameter, actuatable by the user, programmable by a professional ophthalmologist or a combination of the two; with the object of establishing a variable filtering of the spectral transmittance in the vision of a user of the device.

2. DEVICE FOR ASSISTING AND PROTECTING VISION, according to claim 1, **characterized in that** the variable filtering is established for the absorption of the short wave lengths in at least the 400 to 550 nm band.

3. DEVICE FOR ASSISTING AND PROTECTING VISION, according to claim 1 or 2, **characterized in that** said assisting device is achieved optionally combined with an standard optical correction element such as some prescription glasses.

4. DEVICE FOR ASSISTING AND PROTECTING VISION. according to claim 3. **characterized in that** the assisting device consists of a series of supplements that are coupled to these prescription glasses.

5. DEVICE FOR ASSISTING AND PROTECTING VISION, according to claim 4, **characterized in that** the electronic device (12) is included in one of the sidepieces (2) of a glasses body (1) wherein the areas of the frame that are in front of the eyes of the user include the referred electrochromic filters (3, 4).

6. DEVICE FOR ASSISTING AND PROTECTING VISION, according to any one of the previous claims, **characterized in that** the electronic device (12) presents supply means and has a D/A converter (6) the output of which is connected to the electrochromic filters (3, 4), while to its input a memory (5) and a control circuit (7) are connected which through an A/D converter (9) connects with an external potentiometer (8) and through a connection module (10) is coupled to an external connector or terminal (11); such that this terminal (11) forms the referred programmable means and the potentiometer (8) forms the above-mentioned means actuatable by the user.

7. DEVICE FOR ASSISTING AND PROTECTING VISION, according to claim 6, **characterized in that** said memory (5) is an EPROM memory, the control circuit (7) is a PIC circuit or a general purpose microcontroller, the connection module (10) is RS-232-type and the external terminal (11) is USB-type.

8. DEVICE FOR ASSISTING AND PROTECTING VISION, according to claim 6, **characterized in that** the supply means consist of a rechargeable battery located in a belt of the user and cable-connected to the rest of the electronic device (12), or else on a button cell located in the frame of the corresponding glasses (1),

9. DEVICE FOR ASSISTING AND PROTECTING VISION, according to claim 6, **characterized in that** the external potentiometer (8) consists of a variable resistor of sliding or rotating control, or else of two buttons with associated electronic circuitry respectively facilitating an increase and decrease of the filtering carried out by the electrochromic filters (3. 4).

10. DEVICE FOR ASSISTING AND PROTECTING VISION, according to claim 6, **characterized in that** said external terminal (11) forming the programmable means is capable of being complemented or substituted by infrared or radiofrequency wireless programming means applicable to the control circuit (7).

11. DEVICE FOR ASSISTING AND PROTECTING VISION, according to any one of the previous claims, **characterized in that** it has feedback means applied to the electronic device (12) and determined by photodiodes located in front and behind the electrochromic filters (3, 4).

12. DEVICE FOR ASSISTING AND PROTECTING VISION, according to claim 11, **characterized in that** it has a programming distinguishing the type of environmental illumination using the signs of the referred photodiodes, consequently acting on the electrochromic filters (3, 4).

13. DEVICE FOR ASSISTING AND PROTECTING VISION, according to any one of the previous claims, **characterized in that** it has some voltage wave forms exciting the electrochromic filters (3, 4) specifically adapted to its operation, continuous or alternate, these being square with variable work cycle or triangular or sinusoidal, with the object of optimizing the current draw of said filters (3, 4).

14. DEVICE FOR ASSISTING AND PROTECTING VISION, according to any one of the previous claims, **characterized in that** the referred control means are capable of being achieved in such a way that they independently act on the left electrochromic filter (3) and on the right electrochromic filter (4), or else in such a way that they act jointly on both of them (3, 4).
